# EUROPEAN PATENT APPLICATION

(11) **EP 4 122 463 A1**
(43) Date of publication of application: **25.01.2023**
(21) Application number: 21771581.2
(22) Date of filing: 24.02.2021
(51) Int. Cl.: A61K 31/405, A61K 47/40, A61P 15/00

(54) **DRUG IN THE FORM OF A COMPLEX OF 3,3'-DIINDOLYLMETHANE WITH Beta-CYCLODEXTRIN**

(30) Priority: 18.03.2020 RU 2020111302
(71) Applicant: Kiselev, Vsevolod Ivanovich, Moscow 117218 (RU)
(72) Inventor: Kiselev, Vsevolod Ivanovich, Moscow 117218 (RU)
(74) Representative: Ahrens, Gabriele
(86) International application number: PCT/RU2021/000077
(87) International publication number: WO 2021/188012

(57) **Abstract**

The invention relates to the field of medicine and the pharmaceutical industry, and can be used for production of drug forms of 3,3'-diindolylmethane and their use for treatment and prevention of diseases of a female reproductive system. A drug based on 3,3'-diindolylmethane contains β-cyclodextrin as a complexing component in the following ratio of components, wt.%: 3,3'-diindolylmethane 10-20, β-cyclodextrin 80-90. The drug may be in the form of capsules, or suppositories, or tablets, or powder for dissolution in water. A method for production of the drug based on 3,3'-diindolylmethane is that β-cyclodextrin is dissolved in water to obtain a solution concentration of 10-25% based on dry β-cyclodextrin. To the obtained solution 3,3'-diindolylmethane is added at the above mass ratio. The solution is stirred, the resulting suspension is spray dried at a gas temperature of 180 ° C, and the resulting dry product is sieved through a mesh to obtain a drug in powder form. A method of treating cervical neoplasia associated with human papillomavirus (HPV) is that the patient is administered once a vaccine against human papillomavirus, and then the patient is treated locally with a drug containing 3,3'-diindolylmethane in combination with β-cyclodextrin in the form of a vaginal tablet. The invention improves the water solubility and bioavailability of 3,3'-diindolylmethane, improves stability of the chemical composition of the drug, as well as eliminates HPV and eliminates burning sensation and discomfort during local treatment with vaginal tablets.

## Description

### Field of invention

The invention relates to the field of medicine and the pharmaceutical industry, and can be used for production of drug forms of 3,3'-diindolylmethane and their use for treatment and prevention of diseases of the female reproductive system.

### Background of invention

The use of 3,3'-diindolylmethane (DIM) as a drug substance is widely known. The synthesis of 3,3'-diindolylmethane is based on oligomerization of indole-3-carbinol in an acidic medium. The source of indole-3-carbinol is natural products: broccoli, cauliflower, white cabbage, kohlrabi. Currently, therapeutic properties of 3,3'-diindolylmethane have been discovered in relation to a number of diseases, such as myoma, adenomyosis, thyroid hyperplasia, atopic dermatitis, Crohn's disease. This list is constantly updated with new directions in the therapeutic use of 3,3'-diindolylmethane. In recent years, more than 300 scientific articles in the field of application of 3,3'-diindolylmethane and its derivatives have been published. Preparations based on 3,3'-diindolylmethane have unique properties and have enormous pharmaceutical potential.

One of disadvantages of 3,3'-diindolylmethane is its unpleasant odor characteristic of most indoles, which limits its use in a certain category of patients. The second important limitation of diindolylmethane is its low bioavailability, which prevents achievement of necessary therapeutic concentrations of the active substance in the lesions.

An oral drug form is known, including 3,3'-diindolylmethane, Polysorbate 80 and dimethyl sulfoxide [RU 2666242 C1, publ. 06.09.2018]. Solubility of 3,3'-diindolylmethane in DMSO is 18-20%. In this form, proportion of DMSO is not more than 1 % of the 3,3'-diindolylmethane mass - the main active therapeutic substance. In this case, DMSO is used as an additive that improves bioavailability. DMSO is a low-toxic substance; however, its ability to quickly penetrate biological membranes can lead to toxic substances being taken along with the active drug substance. There are examples, when applying DMSO in doses of 606 mg / kg, a patient developed nausea, vomiting, and impaired liver function. In another case, application of DMSO on a woman's skin of 1800 mg / kg caused shortness of breath, cyanosis and disturbances in a testimony of a blood test [I.D.Gulyakin, N.A.Oborotova, V.M.Pechennikov, Solubilization of hydrophobic antitumor drugs, Chemical and Pharmaceutical Journal Volume 48, No. 3 (2014) p. 46-50].

Inhalation drug form for the treatment of diseases of the upper respiratory tract is known, where ethyl alcohol and glycerin are used as a solvent of 3,3'-diindolylmethane in a ratio of 2: 1 [RU 2665974 publ. 05.09.2018]. It is known that alcohol-glycerin mixture is a universal solvent for many organic compounds, including 3,3'-diindolylmethane. In said pharmaceutical form, 0.85-17 mg of 3,3'-diindolylmethane is dissolved in one milliliter of the alcohol-glycerin mixture. When 3,3'-diindolylmethane perorally administered, such form is not suitable, since in order to achieve the necessary therapeutic dose of 3,3'-diindolylmethane (150-300 mg), a patient will have to consume more than 15 ml of ethyl alcohol daily for 2-3 months.

A drug, containing as an active principle 3,3'-diindolylmethane for the treatment of hyperplastic and inflammatory diseases of man is known, it is a solution containing as a carrier a mixture of fish oil and polysorbate, [RU 2419426 C1 publ. 07.05.2011]. The use of fish oil as an auxiliary substance in the active drug form has several advantages: 3,3'-diindolylmethane is highly soluble in oils, and fish oil itself contains polyunsaturated fatty acids omega-3 and omega-6, as well as vitamins A and D. However, the use of fish oil has several serious disadvantages of applying. The main disadvantage of fish oil is its tendency to oxidize and change its physico-chemical composition, which, in turn, can lead to accumulation of toxic substances in its composition, increase acidity, and decrease solubility of the active drug form and leads to formation of sediment. In clinical practice, a course of taking the fish oil, as a rule, does not exceed 30 days, and significant therapeutic effect of using the drug forms based on 3,3'-diindolylmethane occurs in 2-3 months. As a result, a patient receives an excessive dose- response relationship of fish oil.

It is extremely important to note that in numerous *in vitro* studies, biological effects of DIM were strictly dose-dependent. However, in *in vivo* experiments and clinical studies, increasing dosages only in a limited range enhanced effect of this indole. Most authors attribute this to lack of bioavailability of DIM and its low absorption in a stomach. In this regard, development of local drugs based on DIM is very promising.

Earlier, we developed a drug in a form of vaginal suppositories for treatment of cervical neoplasia, which contains 3,3'-diindolylmethane as an active substance and a lipophilic base containing solid confectionery fat, polyvinylpyrrolidone and butylhydroxyanisole (RU 2318510 C1, publ. 10.03.2008). Clinical studies have shown that DIM in the form of vaginal suppositories has a pronounced therapeutic effect and causes regression of cervical neoplasias associated with human papilloma viruses. However, with the widespread use of the drug in clinical practice, two significant drawbacks were discovered. The drug must be stored at a temperature of + 4 ° C, because when stored at room temperature, a discoloration was observed due to decomposition of DIM in the composition of suppositories. In addition, the drug should be prescribed twice a day, this is necessary to achieve a therapeutic concentration of DIM, which is not always convenient for patients. An increase in concentration of the active substance in suppositories leads to their friability and significant reduction in shelf time.

Medicines are known in which non-toxic cyclodextrins are used as a means of increasing the water solubility of an organic compound.

US Patent No. 5,914,122 discloses the use of cyclodextrins and their derivatives, such as β-CD, γ-CD or HP-β-CD, for preparation of aqueous budesonide solutions. The disadvantage of this form is its low physico-chemical stability in the liquid state. It is reported that the shelf time of the pharmaceutical form is from 3 to 6 months.

Closest to the proposed drug and method for its preparation are the self-emulsifying composition adopted for the prototype, including 3,3'-diindolylmethane, a mixture of natural oils, surface active agent, additional surfactants and a solvent, as well as a method for its preparation, which includes following stages: (a) mixing a solvent, a surfactant with an HLB number greater than 7, and an auxiliary surfactant with an HLB number equal to or less than 7 (and optionally additional excipients such as polycarbonate and / or poloxamer), (b) heating and stirring the mixture to homogeneity, (c) cooling the mixture (for example, to about 50 ° C or from 40 to 60 ° C) and adding diindolylmethane, and optionally one or more additional active ingredients, and (d) further mixing the mixture until uniform. The resulting mixture can be made in a suitable drug form, such as soft or hard gelatin capsules, and be allowed to cool further to ambient temperature [US 9918965 B2, 20.03.2018].

A disadvantage of this composition is possible hydrolysis of lipid substances with the formation of monoglycerides, which leads to decrease in dissolving ability of the composition with respect to 3,3'-diindolylmethane, and, as a result, precipitation of 3,3'-diindolylmethane in the form of a precipitate in a gastrointestinal tract [Kaukonen A.M., Boyd B.J., Porter C.J., Charman W.N. Drug solubilization behavior during in vitro digestion of simple triglyceride lipid solution formulations, Pharm. Res. (2004) N 21. P. 245-253]. The patent materials do not contain information on the behavior of the composition in the presence of enzymes that change the chemical compound of the composition, thereby reducing solubility of the active pharmaceutical ingredient in the system [Yu.A. Baskakova. Justification and development of technology for a concentrate of polyunsaturated fatty acids omega-3 from fish fats // Ph.D. thesis. Moscow (2017) p. 322]. High content of surfactants in the pharmaceutical composition can cause irritation of a gastrointestinal tract [K.V. Alekseev, K.G. Turchinskaya, E.V. Blynskaya, R.V. Tikhonova. Technology of self-emulsifying drug delivery systems, Bulletin of New Medical Technologies. V.2, No. 1, p. 128]. In addition, in the studies conducted [Wang Z, Klipfell E, Bennett BJ, Koeth R, Levison BS, Dugar B, Feldstein AE, Britt EB, Fu X, Chung YM, Wu Y, Schauer P, Smith JD, Allayee H, Tang WH, DiDonato JA, Lusis AJ, Hazen SL Gut flora metabolism of phosphatidylcholine promotes cardiovascular disease. Nature. (2011) 472 (7341): 57-63] it was shown that the phosphatidylcholine adjuvant used in the composition can lead to growth of atherosclerosis.

There is a method of treating squamous intraepithelial lesions of a cervix, independent of their association with HPV, including the use of vaginal suppositories "Cervicon-DIM" containing 100 mg diindolylmethane, as well as excipients: solid fat, crospovidone and dye, while the drug is used for not less than 6 months at a dosage of 200 mg / day of diindolylmethane [RU 2552332 C1, publ. 10.06.2015].

A disadvantage of this method is that when prescribing the drug, patients quite often complain of burning and discomfort, which is probably due to rapid release of the active substance.

Closest to the proposed is a method of combination therapy for cervical dysplasia associated with human papillomavirus, which consists in the fact that they carry out local treatment of a patient with a drug using, namely, vaginal suppositories containing 3,3'-diindolylmethane, in this case, before starting local treatment, a patient is once administered a vaccine against human papilloma virus (RU 2701525 C1, publ. 27.09. 2019).

This method has high therapeutic efficacy. However, when prescribing the drug in this case, patients also quite often complain of burning and discomfort. In addition, in the study of combination therapy, including vaccination and the use of suppositories containing 3,3'-diindolylmethane, a rather rapid regression (within 2 months from the start of treatment) of foci of dysplasia was observed according to colposcopy and histological analysis. However, with these follow-up periods, it was not possible to eliminate high-risk HPV. This fact indicates a high probability of recurrence, which also requires its solution.

Thus, despite the promise of this approach, the drug needs to be optimized, in particular, the possibility of storage at room temperature, which will greatly simplify logistics problems. It is necessary to increase content of the active substance and ensure gradual release of the substance. These decisions should shorten treatment time and eliminate the side effects of burning when prescribing the drug.

### Disclosure of the invention

The present invention is based on the technical problem of creating a medicinal product having a high solubility level of 3,3'-diindolylmethane in an aqueous solution and masking unpleasant odor of indole derivatives, creating a method of preparation of said drug, ensuring stability of the drug for a long time, as well as creating a method of treating cervical neoplasia in women, in which there is no itching and burning, and HPV elimination is also provided.

The technical result of the invention is to increase the solubility of 3,3'-diindolylmethane in water in concentrations from 1 mg / ml and mask the unpleasant odor of the main ingredient.

The technical result is achieved due to the fact that a drug based on 3,3'-diindolylmethane containing 3,3'-diindolylmethane and a complexing component differs from the prototype in that the complex contains β-cyclodextrin as a complexing component in the following ratio of components wt.% (or mass %):
3,3'-diindolylmethane 10-20
β-cyclodextrin 80-90
The drug may be in the form of capsules, or suppository, or tablets, or powder for dissolution in water.

The technical result also consists in providing a drug with a stable composition.

This technical result is achieved by a method of creating a drug based on 3,3'-diindolylmethane, which consists in dissolving β-cyclodextrin in water to obtain a solution concentration of 10-25% based on dry β-cyclodextrin, and adding 3,3'-diindolylmethane to the obtained solution, with the following ratio of 3,3'-diindolylmethane and dry β-cyclodextrin, wt. %:
3,3'-diindolylmethane 10-20
β-cyclodextrin 80-90,
the solution is stirred, the resulting suspension is spray dried at a gas temperature of 160-185 ° C, the resulting dry product is sieved to obtain a drug in powder form.

Also, a technical result is to ensure the elimination of HPV, as well as to eliminate the side effects of burning and discomfort during local treatment with a drug based on 3,3'-diindolylmethane.

The technical result is achieved by a method of treating cervical neoplasia associated with human papillomavirus, which consists in the fact that a patient is administered once a vaccine against human papillomavirus, after which local therapy of the patient is carried out using a drug containing 3,3'-diindolylmethane, the method differs from the prototype in that a complex of 3,3'-diindolylmethane and β-cyclodextrin in the form of a vaginal tablet is used as a drug.

### List of illustrations

**FIG. 1** shows the structure of a complex of 3,3'-diindolylmethane and β-cyclodextrin.
**FIG. 2** is a graph of maximum solubility of diindolylmethane versus β-cyclodextrin concentration.

### Examples of the invention

The main active component of the declared composition is 3,3 "-diindolylmethane. As an additional substance, the proposed medical preparation includes β-cyclodextrin, which has the ability to increase solubility of many organic substances.

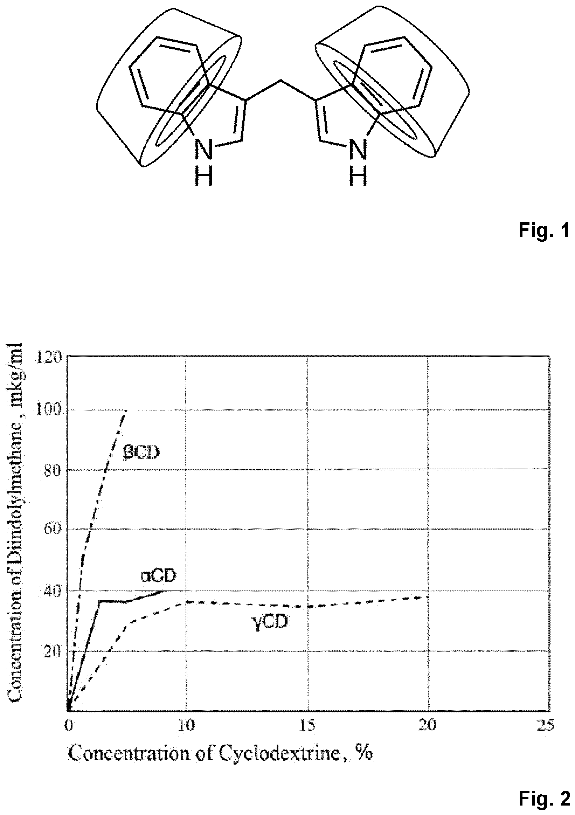

It was found that increase in solubility of organic substances in the presence of cyclodextrins occurs due to formation of a stable complex without formation of covalent bonds. FIG. 1 schematically shows the structure of the complex of 3,3'-diindolylmethane and a cyclodextrin. The cyclodextrin is shown as two rings. An inner part of the cyclodextrin structure has hydrophobic properties, and an outer part is hydrophilic. Due to these unique properties of cyclodextrin, solubility of certain organic substances is ensured.
- Cyclodextrins are widely used in various medicines: Nicorette, Zyrtek, Ulgut, Voriconazole, Kyprolis, Nexterone and several others.
- In the above medicines, cyclodextrin not only helps to increase their solubility and bioavailability of the active substance, but also improves organoleptic properties (taste and odor) of the drug.

Solubility of 3,3'-diindolylmethane was investigated as a function of concentration of various cyclodextrins. The following were used in the experiments:
- α-cyclodextrin (αCD);
- β-cyclodextrin (βCD);
- γ-cyclodextrin (γCD).
Results of experiments to determine the solubility in solutions of various cyclodextrins are shown in figure 2. The results showed that the maximum solubility of 3,3'-diindolylmethane is achieved in combination with β-cyclodextrin (βCD).

Solutions of a complex of 3,3'-diindolylmethane with various cyclodextrins turned out to be thermodynamically unstable. A day later, pH of the solutions decreased from 7.3-7.8 to 6.7-7.1. The thermodynamic stability of the complex was achieved due to conversion of the complex from solution to crystalline form. For this, the complex obtained in an aqueous solution was spray dried. The crystalline powder obtained by drying was sieved through nested 1 mm sieves.

The invention is illustrated by the following examples.

### Example 1.

### A method of obtaining a drug form of a complex of 3,3'-diindolylmethane and β-cyclodextrin (DIM + βCD).

The powder of β-cyclodextrin in an amount of 5600 g (humidity 12%) was dissolved in 22.6 liters of water, gradually mixing. 713 g of 3,3'-diindolylmethane was added to the β-cyclodextrin solution. The suspension was stirred for 4 hours. The resulting suspension was fed into spray drying at a gas temperature of 180°C. The yield of the obtained diindolylmethane-cyclodextrin complex (DIM + βCD) was 5350 g. The dry product was sieved through nested 1 mm sieves. The ratio of DIM and dry β-cyclodextrin in the resulting product was 12.6 wt.% and 87.4 wt.%, which corresponds respectively to the molar ratio of these components in the complex.

The resulting complex is completely dissolved in water.

The solution of the complex DIM + βCD was subjected to chromatographic analysis. The results of physicochemical analysis showed almost complete coincidence of chromatograms of pure 3,3'-diindolimethane and the DIM + βCD complex.

The complex was also prepared with the following ratios of DIM and dry β-cyclodextrin.
1. DIM 10 wt.%, βCD 90 wt.%
2. DIM 20 wt.%, βCD 80 wt.%

Deviation of the ratio from the molar within the specified limits and a small excess of one or another component in the resulting product does not affect properties of the drug.

### Example 2.

### Assessment of organoleptic properties of the drug form

A group of 10 patients taking the drug based on 3,3'-diindolylmethane as prescribed by a doctor was asked to evaluate organoleptic properties of the new complex (taste and odor). All participants noted significant positive effect on taste and masking effect of cyclodextrin on the unpleasant odor of 3,3'-diindolylmethane.

### Example 3.

### Composition of the drugs.

### 1. Vaginal tablets, composition:

Complex DIM + βCD - 99%
Magnesium stearate - 1%.

The tablets may be coated to facilitate insertion into the vagina. Vaginal tablets can also be made with other known excipients or can be obtained by simply compressing the substance without using auxiliary components.

### 2. Oral tablets, composition:

Complex DIM + βCD - 71%
Microcrystalline cellulose - 21%;
Sodium starch glycolate - 7%;
Magnesium stearate - 1%.

### Tablets options:

1) For resorption, either chewable, without a coating.
2) Prolonged or regular release, both without a coating and with an enteric coating.

### Example 4.

**Study of stability of vaginal drug forms containing 3,3'-diindolylmethane.** Table 1 shows the results of a study of stability of vaginal drug forms containing 3,3'-diindolylmethane when stored at 25 ° C for 6 months.

**Table 1**

| Parameters Drug form | Reference color | Color after 6 months of storage | The content of the active substance DIM original | The content of the active substance DIM after storage |
|---|---|---|---|---|
| Vaginal suppositories Composition: DIM - 100 mg, lipophilic base | Light pink | Light brown | 100 mg | 80 mg |
| Vaginal tablets Composition: DIM - 200 mg, β-cydodextrin, magnesium stearate | White | White | 200 mg | 200 mg |

Conclusion: the drug form DIM + βCD is stable during prolonged storage at a temperature not exceeding 25 ° C.

### Example 5.

### Study of concentration of 3,3'-diindolimethane in the blood plasma of patients.

In clinical studies, effectiveness of a water-soluble complex based on cyclodextrins and crystalline 3,3'-diindolylmethane in equal doses was compared. In all studied groups of patients, pronounced positive dynamics were found when taking water-soluble complexes and the absence of such when taking crystalline 3,3'-diindolylmethane. The results of measuring the concentration of 3,3'-diindolylmethane in the plasma of patients are presented in table 2.

**Table 2**

| Drug | DIM dosage, mg | The concentration of DIM in plasma, ng / ml |
|---|---|---|
| DIM crystalline | 10 | not determined |
| DIM+ βCD | 10 | more than 100 |
| DIM + RAMEB* | 10 | more than 100 |
| DIM+SBEβCD** | 10 | more than 100 |
| DIM crystalline | 100 | less than 30 |
| DIM+ βCD | 100 | more than 300 |
| DIM + RAMEB | 100 | more than 300 |
| DIM+SBEβCD | 100 | more than 300 |

| | | |
|---|---|---|
| * DIM + RAMEB - 3,3'-diindolylmethane + methylated β-cyclodextrin ** DIM + SBEβCD - 3,3'-diindolylmethane + sulfobutyl ether β-cyclodextrin | | |

The test results showed that stable bioavailability parameters were achieved using water-soluble complexes DIM + βCD, DIM + RAMEB and DIM + SBEβCD at a dosage of 100 mg per day. In this case, the plasma DIM concentration when taking 100 mg of water-soluble complexes was more than 300 ng / ml.

### Example 6.

### Treatment of cervical neoplasia with vaginal tablets containing 3,3'-diindolylmethane (200 mg) in combination with β-cyclodextrin.

The main criteria for the inclusion of patients in a clinical study.
1. Colposcopic picture of lesions of the cervix against the background of the transformation zone of type I and II
2. Age 18-45 years.
3. The diagnosis of cervical intraepithelial neoplasia 1-2 degrees (CIN I-II), verified histologically.
4. The presence of human papillomavirus (HPV) of 16 or 18 types identified by polymerase chain reaction.

According to the main demographic indicators (age, height, body weight, body mass index), the study groups did not differ, which made it possible to combine them for analysis according to the research objectives/ Significant differences in characteristics of the initial state: data of a gynecological history (age, menarche, menstruation, interval between menstruation, age of sexual debut) were not detected.

Treatment regimen included a single vaccination with Gardosil or Cervarix and, from the next day, the administration of vaginal tablets containing 3,3'-diindolylmethane (200 mg) in combination with β-cyclodextrin overnight for two months.

After completion of the treatment period, the patients underwent colposcopic examination and cytological analysis of the cervix and examination for HPV types 16 and 18 by PCR. Evaluation of the effectiveness of therapy after 2 months of treatment - according to colposcopy, cytological examination and elimination of HPV DNA according to the results of PCR are shown in table 3.

**Table 3**

| | | DIM+ βCD | | DIM+ βCD + Gardosil | | DIM+ βCD + Cervarix | |
|---|---|---|---|---|---|---|---|
| | | Number of patients | % in Group | Number of patients | % in Group | Patient | % in Group |
| Effect: normalization according to colposcopy and cytological examination | No | 5 | 50 | 2 | 20 | 1 | 10 |
| | Yes | 5 | 50 | 8 | 80 | 9 | 90 |
| HPV DNA Elimination | No | 8 | 80 | 3 | 30 | 2 | 20 |
| | Yes | 2 | 20 | 7 | 70 | 8 | 80 |

As follows from the results presented in table 3, the combination of vaccine preparations with the vaginal tablets containing 3,3'-diindolylmethane shows a pronounced therapeutic effect compared with monotherapy with tablets. Elimination of HPV should be considered a particularly important result of healing properties of the vaginal tablets, which was not achieved in the previous work with vaginal suppositories [RU 2701525 C1 publ.27.09.2019]. Achieving this effect with the help of the vaginal tablets containing the DIM + βCD complex allows to avoid recurrence of dysplastic processes. It should also be noted that when prescribing the vaginal tablets, the patients did not complain of burning and discomfort.

## Claims

1. A drug based on 3,3'-diindolylmethane containing 3,3'-diindolylmethane and a complexing component, **characterized in that** as the complexing component it contains β-cyclodextrin in the following ratio, wt.%:
| | |
|---|---|
| 3,3'-diindolylmethane | 10-20 |
| β-cyclodextrin | 80-90 |

2. The drug according to claim 1, **characterized in that** it has the form of capsules, or suppositories, or tablets, or powder for dissolution in water.

3. A method of obtaining a drug based on 3,3'-diindolylmethane, which consists in dissolving β-cyclodextrin in water to obtain a solution concentration of 10-25% based on dry β-cyclodextrin, then 3,3'-diindolylmethane is added to the obtained solution in the following ratio of 3,3'-diindolylmethane and dry β-cyclodextrin, wt.%:
| | |
|---|---|
| 3,3'-diindolylmethane | 10-20 |
| β-cyclodextrin | 80-90, |
the solution is stirred, the resulting suspension is spray dried at a gas temperature of 160-185 ° C, the resulting dry product is sieved to obtain a drug in powder form.

4. A method of treating cervical neoplasia associated with human papillomavirus, which consists in administering to a patient a single vaccine against human papillomavirus, after which the patient is treated locally with a drug containing 3,3'-diindolylmethane, **characterized in that** the used drug is a complex of 3,3'-diindolylmethane and β-cyclodextrin according to claim 1 in the form of vaginal tablet.
